# EUROPEAN PATENT APPLICATION

(11) **EP 3 909 541 A1**
(43) Date of publication of application: **17.11.2021**
(21) Application number: 21173757.2
(22) Date of filing: 13.05.2021
(51) Int. Cl.: A61B 90/00, A41D 13/11, A61B 90/50

(54) **PATIENT SHIELD**

(30) Priority: 14.05.2020 US 202063024820 P
(71) Applicant: Corona Shield LLC, Milwaukee, WI 53223 (US)
(72) Inventor: SELLARS, William, Milwaukee, WI 53209 (US); PORTER, Douglas, Wilsonville, OR 97070 (US); RODRIGUEZ, Hugo, Portland, OR 97219 (US)
(74) Representative: Forresters IP LLP

(57) **Abstract**

An adjustable patient shield which a protects a user such as a healthcare provider from aerosol and particulate matter emanating from a patient or from equipment.

## Description

### CROSS REFERENCE TO RELATED APPLICATIONS

This application claims priority to U.S. Provisional Application Serial No. 63/024,820, filed May 14, 2020, the entire contents of which is incorporated herein by reference.

### FIELD OF THE INVENTION

The subject matter disclosed herein relates to a patient shield, and more particularly, to a patient shield which a protects a user from aerosol and particulate matter emanating from a patient or from equipment.

### BACKGROUND OF THE INVENTION

Sneezes and coughs from an infected patient can explosively project droplets more than twelve feet where they can be inhaled by a user such as a dentist, hygienist, doctor or other practitioner. The projected droplets can contaminate the room and facility in which the patient is being treated thereby making it difficult to adequately sanitize between patients.

Current vacuum systems used in facilities and similar situations cannot capture droplets and debris emanating from a patient who coughs or sneezes as the velocity of the expectorant is too fast for a vacuum to capture. This is true for aerosol and particulates coming from rotary equipment as well. In an attempt to collect the material emanating from the patient, a simple open hose is often positioned near a patient without any directive structure or apparatus. The velocity of a cough, sneeze or material emanating from a patient or from a tool is too fast to be captured by even the strongest vacuum by itself.

### SUMMARY OF THE INVENTION

The present invention includes a shield for protecting a user from aerosol and particulate matter emanating from a patient or from equipment used on the patient. The shield comprises a transparent barrier adjustably positionable adjacent a patient, an articulating arm secured to the barrier for adjusting the barrier in multiple orientations relative to the patient and a mounting base secured to the articulating arm.

The present invention includes a shield for protecting a user from aerosol and particulate matter emanating from a patient or from equipment. The shield comprises a transparent barrier adjustably positionable adjacent a patient, an articulating arm securable to the barrier to enable the barrier to be adjustable and repositionable in multiple orientations relative to the patient, a vacuum generator for creating a vacuum adjacent the barrier to remove aerosol and particulate matter emanating from the patient or from equipment and a mounting base.

The present invention includes a shield for protecting a user from aerosol and particulate matter emanating from a patient or from equipment. The shield comprises a dome-shaped barrier adjustably positionable adjacent a patient, a segmented articulating arm secured to the barrier and enabling the barrier to be adjustable and repositionable in multiple orientations relative to the patient, a mounting base including locking casters, a vacuum generator supported by the mounting base, a vacuum hose in communication with the vacuum generator and the barrier, a light and a hepa filter.

### BRIEF DESCRIPTION OF THE DRAWINGS

Preferred exemplary embodiments of the invention are illustrated in the accompanying drawings in which like reference numerals represent like parts throughout, and in which:
FIG. 1 is a perspective view of a patient shield of the present invention in use;
FIG 2 is a perspective view of the patient shield in use;
FIG. 3 is a perspective view of the patient shield in use;
FIG. 4 is a perspective view of the patient shield in use;
FIG. 5 is a perspective view of a shell and a bracket of the patient shield;
FIG. 6 is a perspective view of the shell and the bracket;
FIG. 7 is a rear view of the shell and the bracket;
FIG. 8 is a bottom view of the shell and the bracket;
FIG. 9 is an exploded view of the shell and the bracket;
FIG. 10 is a front view of the shell and the bracket;
FIG. 11 is a side view of the shell and the bracket;
FIG. 12 is a top view of the shell and the bracket;
FIG. 13 is a perspective view of the patient shield;
FIG. 14 is a perspective view of the patient shield with an alternate mounting location;
FIG. 15 is a perspective view of the patient shield with an alternate mounting location;
FIG. 16 is a perspective view of the patient shield with an alternate mounting location;
FIG. 17 is an exploded perspective view of a mounting assembly;
FIG. 18 is a partial perspective view of the patient shield in use;
FIG. 19 is a partial perspective view of the patient shield in use;
FIG. 20 is a partial perspective view of the patient shield in use;
FIG. 21 is a partial perspective view of the patient shield in use;
FIG. 22 is a partial perspective view of the patient shield in use;
FIG. 23 is a partial perspective view of the patient shield in use;
FIG. 24 is a partial perspective view of the patient shield in use;
FIG. 25 is a partial perspective view of the patient shield in use;
FIG. 26 is a perspective view of a second embodiment of a patient shield in use;
FIG. 27 is a partial perspective of the second embodiment of the patient shield in use;
FIG. 28 is a top view of a baffle of the second embodiment of the patient shield;
FIG. 29 is a perspective view of the baffle of the second embodiment in use;
FIG. 30 is a perspective view of a third embodiment of a patient shield;
FIG. 31 is a perspective view of the patient shield thereof;
FIG. 32 is side view of the patient shield thereof;
FIG. 33 is front view of the patient shield thereof;
FIG. 34 is a side view of the patient shield thereof;
FIG. 35 is a back view of the patient shield thereof;
FIG. 36 is a perspective view of the patient shield thereof; and
FIG. 37 is perspective view of the patient shield thereof.

Before any embodiments of the invention are explained in detail, it is to be understood that the invention is not limited in its application to the details of constructions and the arrangement of components set forth in the following description or illustrated in the drawings. The invention is capable of other embodiments and of being practiced or of being carried out in various ways.

### DETAILED DESCRIPTION OF THE INVENTION

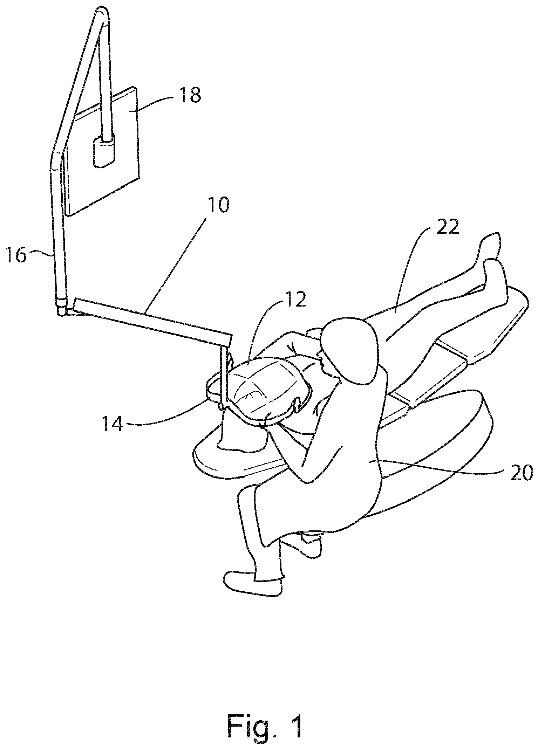

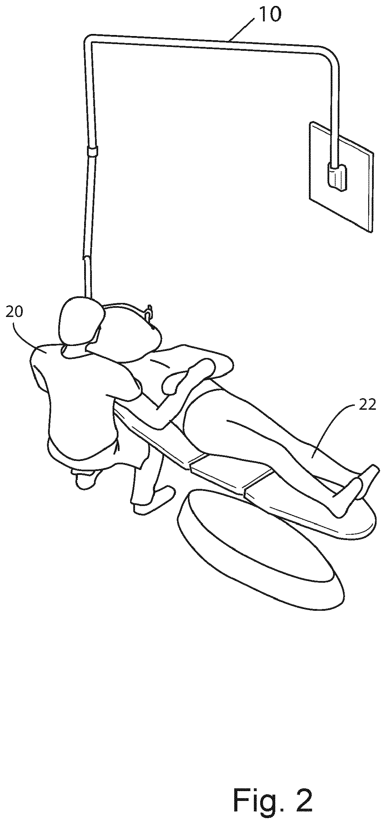

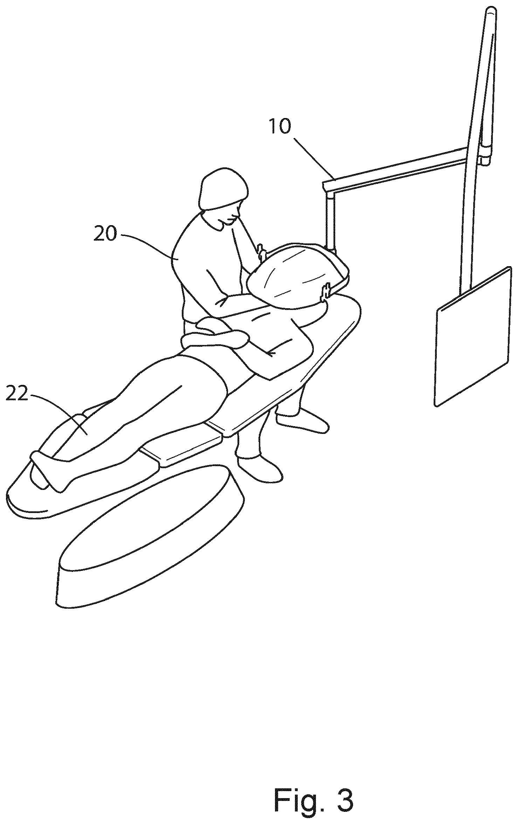

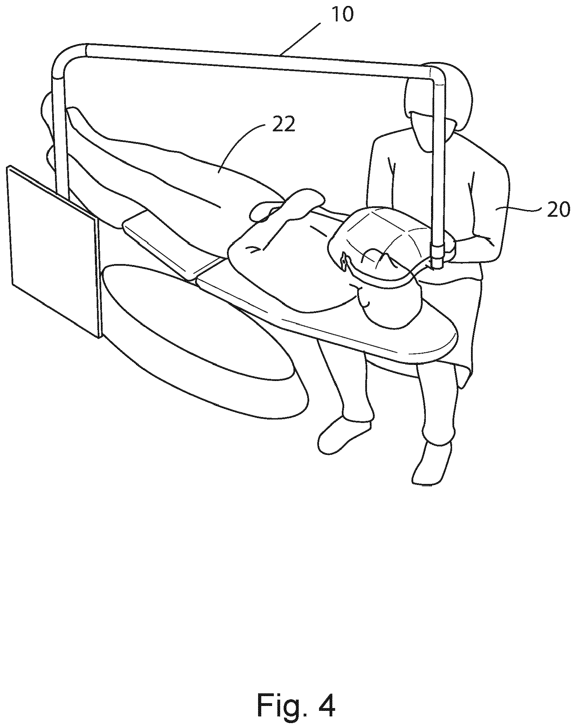

As shown in a first embodiment in Fig. 1, the invention is an adjustable, self-supporting, transparent patient shield 10 which protects a user 20 such as a healthcare or other service provider from biological particulate matter emanating from a patient 22 or from equipment such as dental equipment for example. The shield 10 includes a barrier 12 and a bracket 14 that are suspended by an articulating arm 16 and a mounting assembly 18 from an unobtrusive mounting location. The mounting location could be the ceiling, wall, floor, cabinet, accessory cart, self-contained cart, chair or other nearby structure such as shown in Figs. 2-4. Flexibility of mounting locations and type means any treatment room or facility can be outfitted with the shield 10.

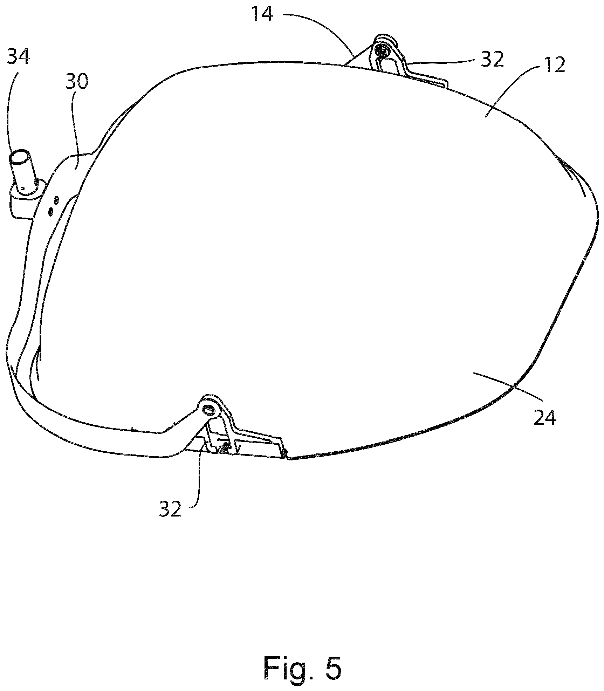

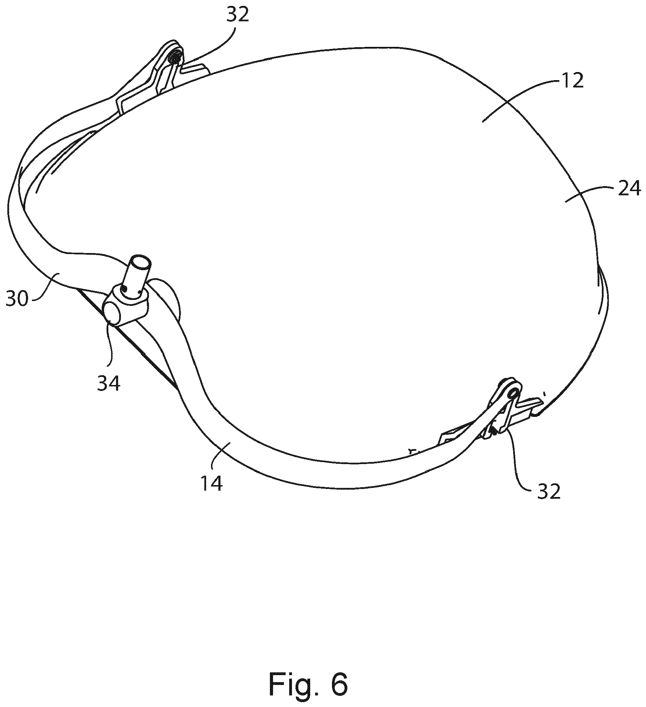

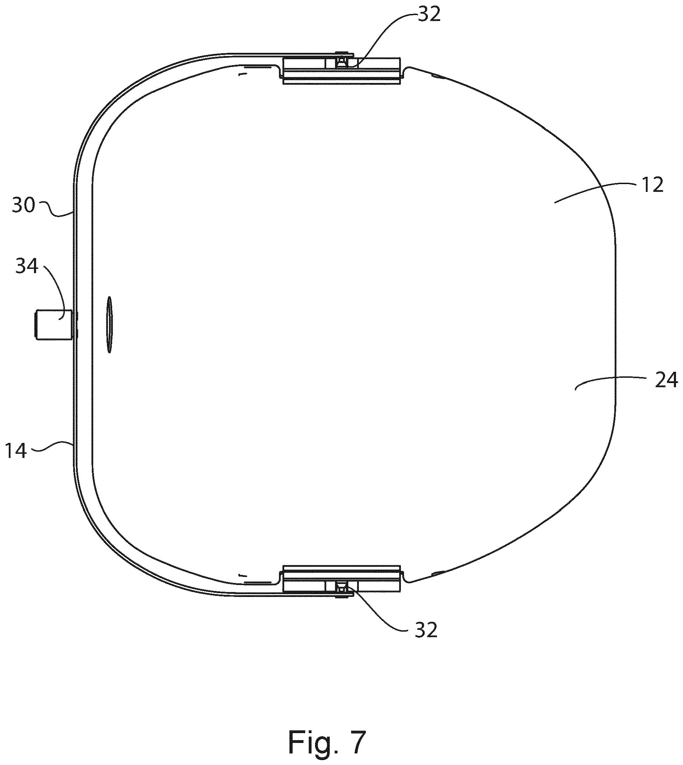

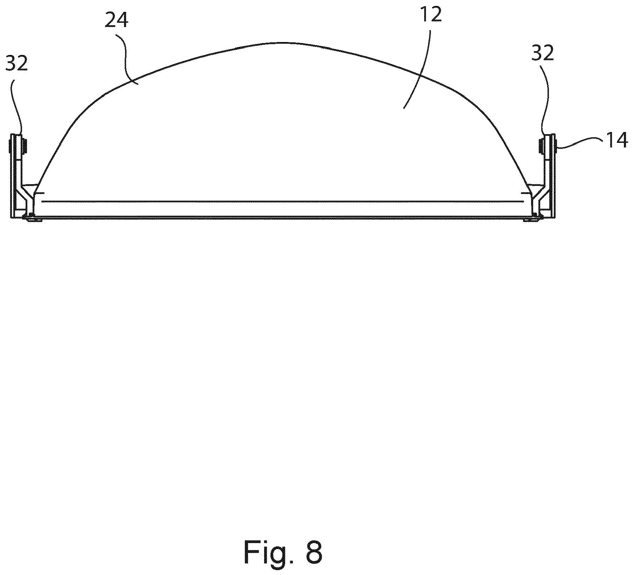

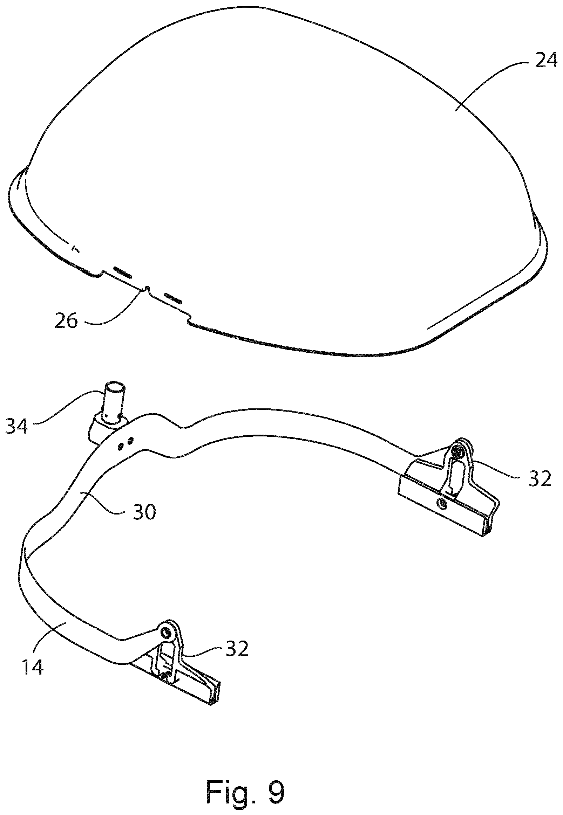

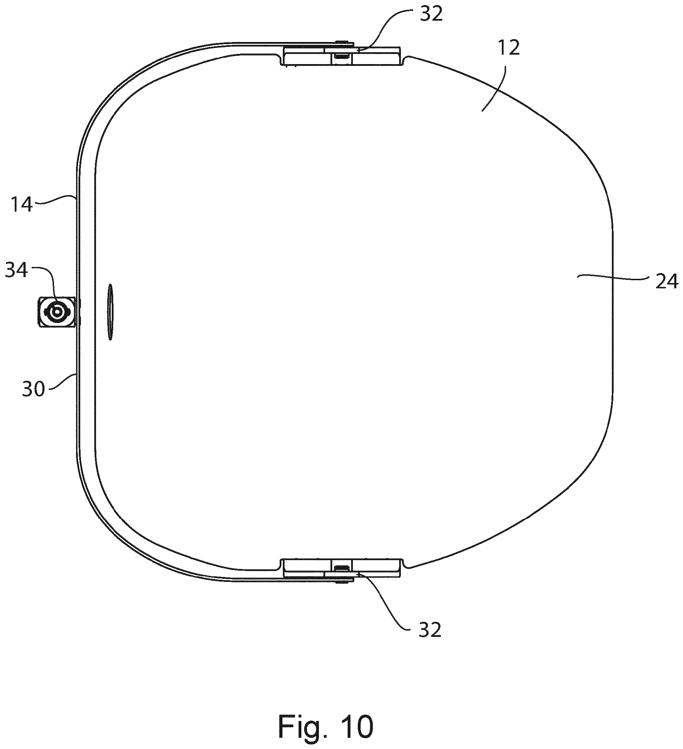

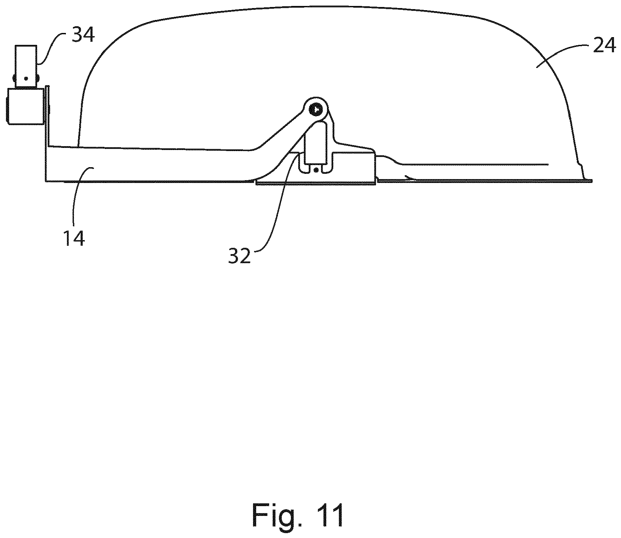

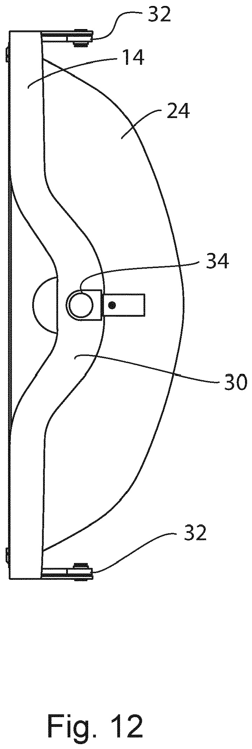

Referring to Figs. 5-12, the shell 12 includes a dome-shaped barrier 24 and a pair of mounting recesses 26 (Fig. 9). The barrier 24 is preferably manufactured from optical grade polycarbonate, however, other materials can also be utilized such as glass, acrylic, other synthetic polymer, screen material or a curtain of air for example. The barrier 24 preferably is dome-shaped, however, other shapes can also be utilized.

The bracket 14 includes a generally u-shaped portion 30, a mounting arm 32 on each end of the u-shaped portion 30 and a mounting arm 34 in the center of the u-shaped portion 30. The bracket 14 is pivotally secured to the shell 12 by connection of the mounting arms 32 that are secured to the mounting recesses 26 of the barrier 24. The bracket 14 is secured to the articulating arm 16 using the mounting bracket 34. However, it should be noted that other ways and methods of securing the barrier 24 to the bracket 14 and the bracket 14 to the articulating arm 16 can also be utilized. Further, the function of the bracket 14 can also be integrated into the shell 12 so that the articulating arm 16 is securable directly to the shell 14.

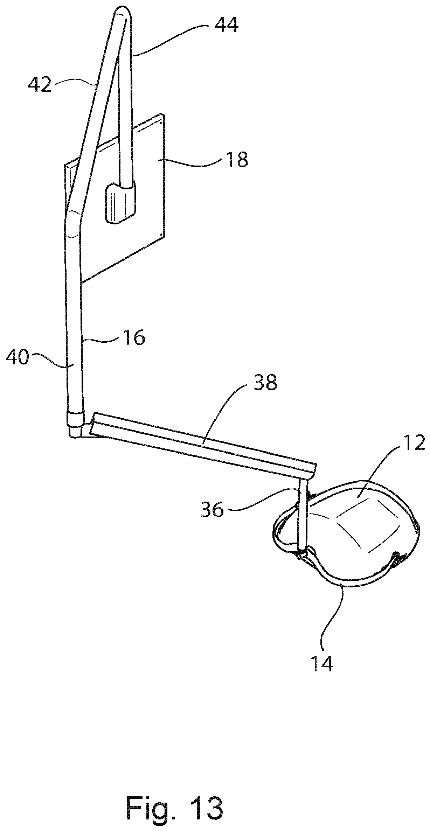

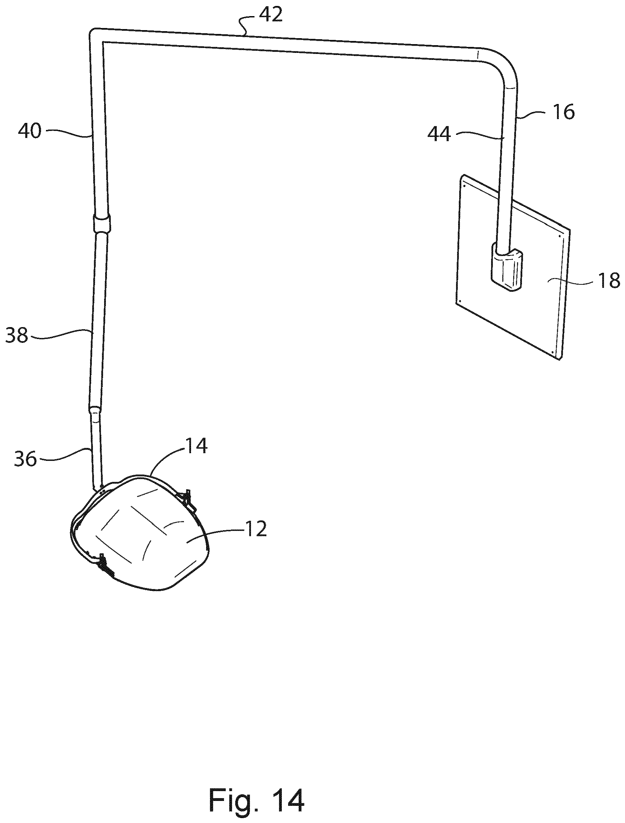

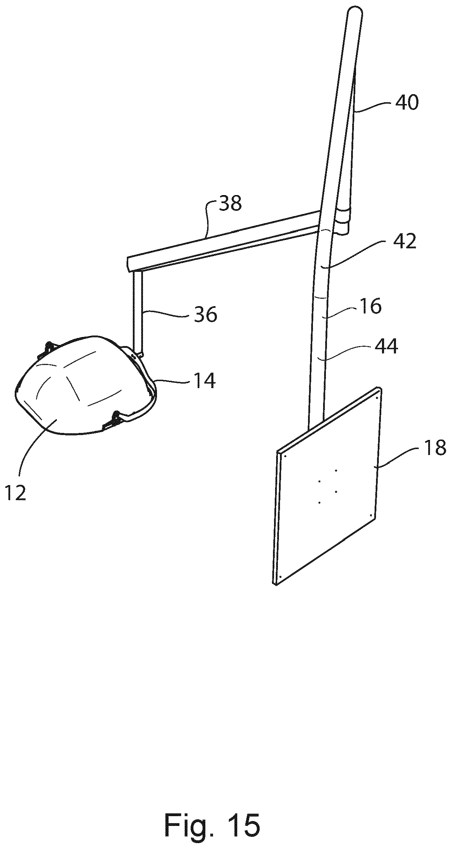

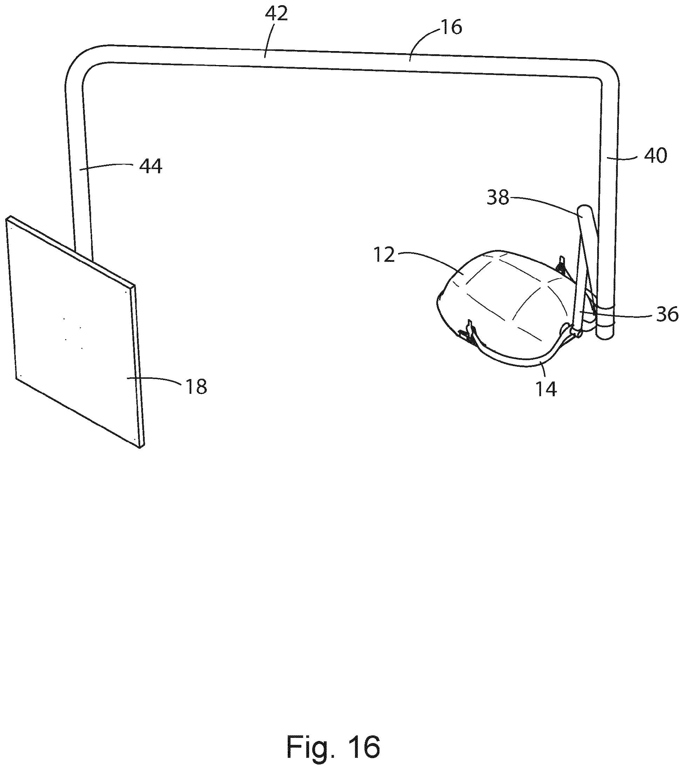

With references to Figs. 13-16, the articulating arm 16 is shown. The articulating arm 16 includes five connected segments 36, 38, 40, 42 and 44 which in combination allow the barrier 24 to be freely moved into multiple orientations. The various figures show the articulating arm 16 in various positions. The articulating arm 16 is preferably manufactured from aluminum, however, other materials can also be utilized such as steel, plastic and titanium for example. It should be noted that other types of articulating arms, non-segmented articulating arms and articulating arms with more or less than five segments can also be utilized.

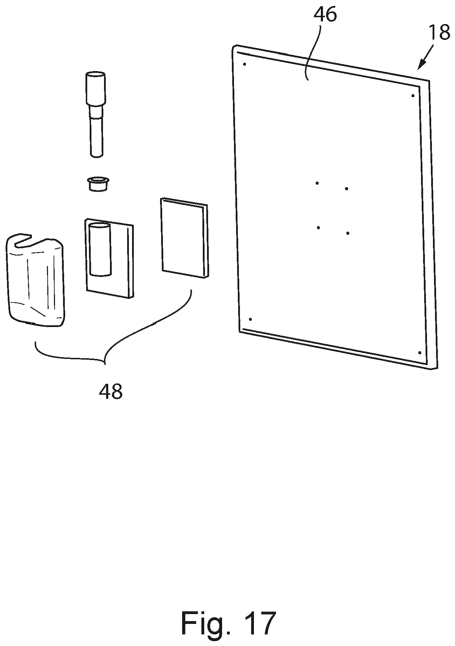

As shown in Fig. 17, the mounting assembly 18 includes a mounting board 46. The mounting board 46 is conventionally securable to the mounting location. Segment 44 of the articulating arm 16 is moveably securable to the mounting board 46 via mounting components 48. The shield 10 can be easily moved by the user 20 using one hand and can be moved anywhere within a volume of space where it can be positioned between the user 20 and the patient 22. Single handed operation allows rapid repositioning of shield 10 during procedures. Alternatively, the shield 10 could be repositioned with an electric motor or pneumatically.

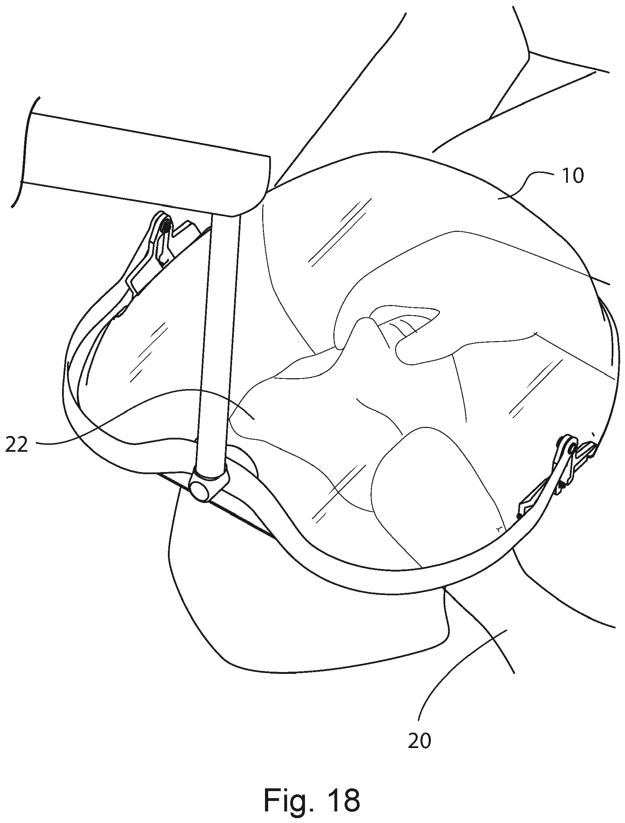

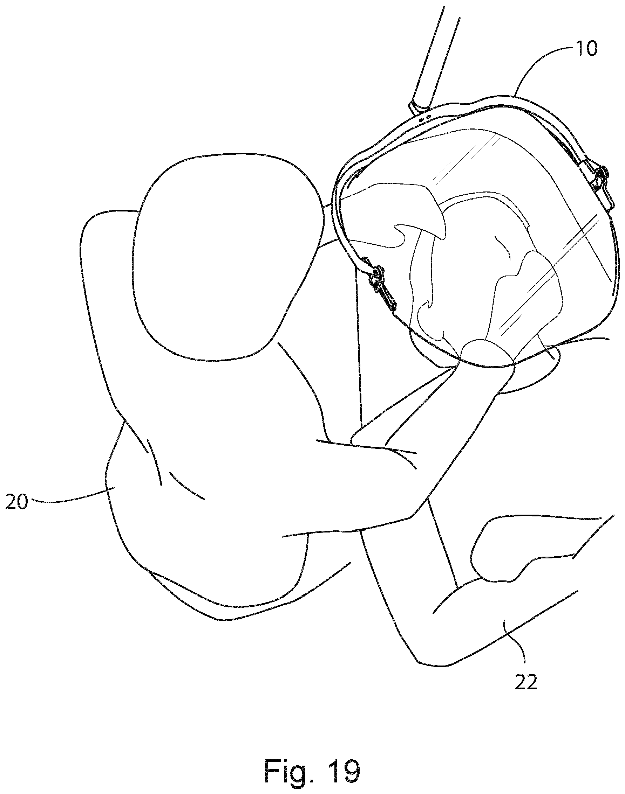

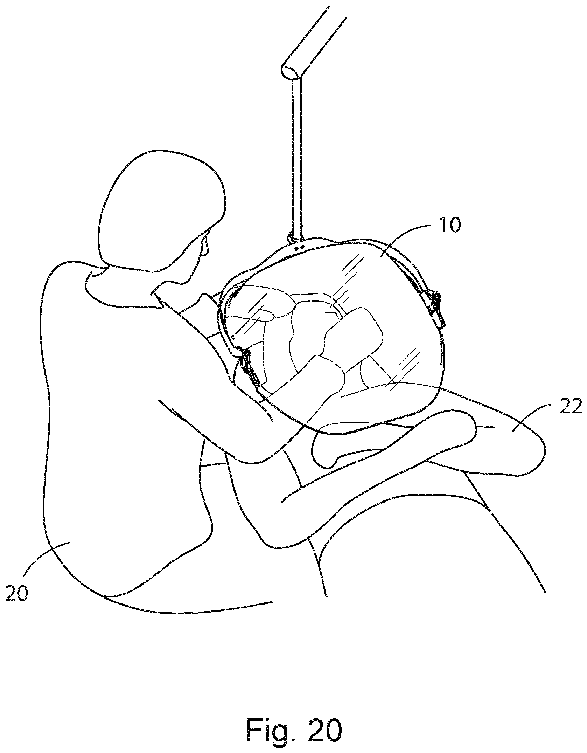

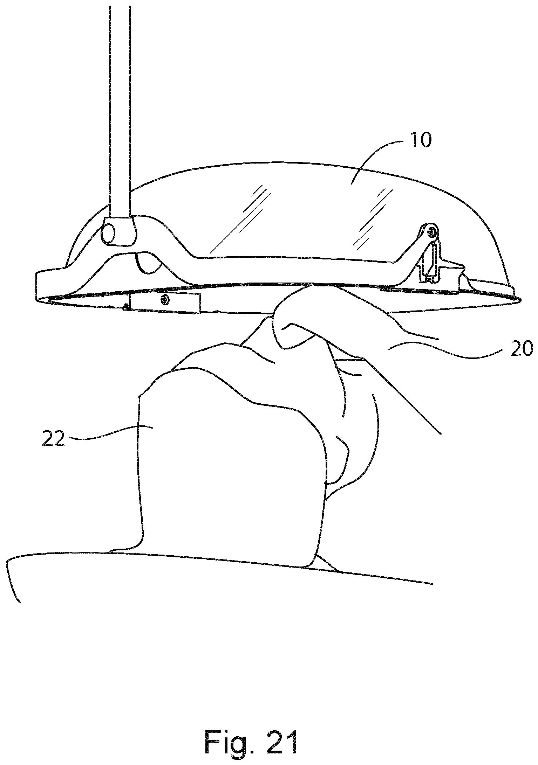

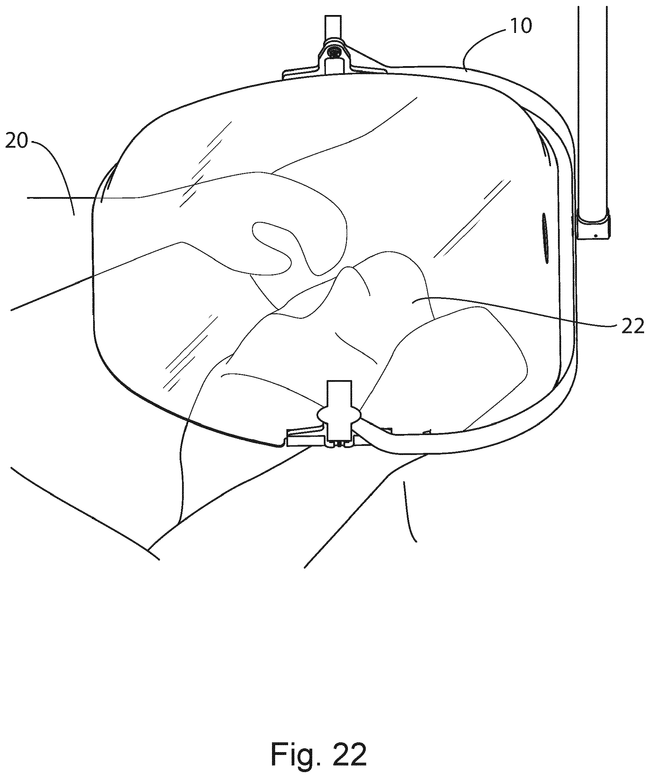

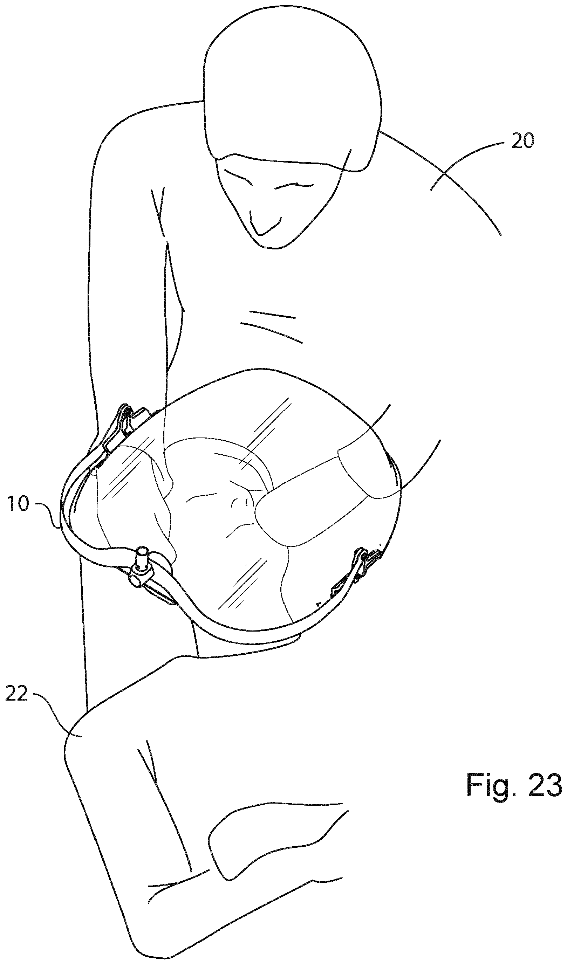

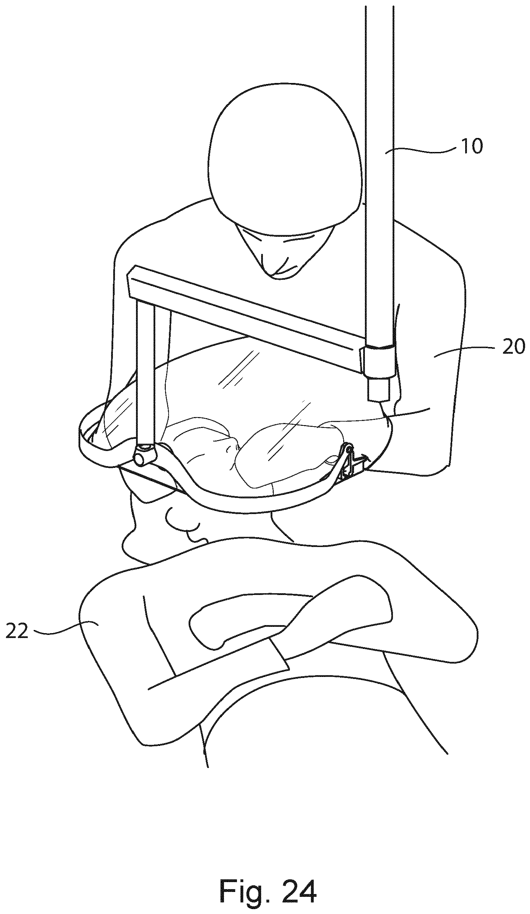

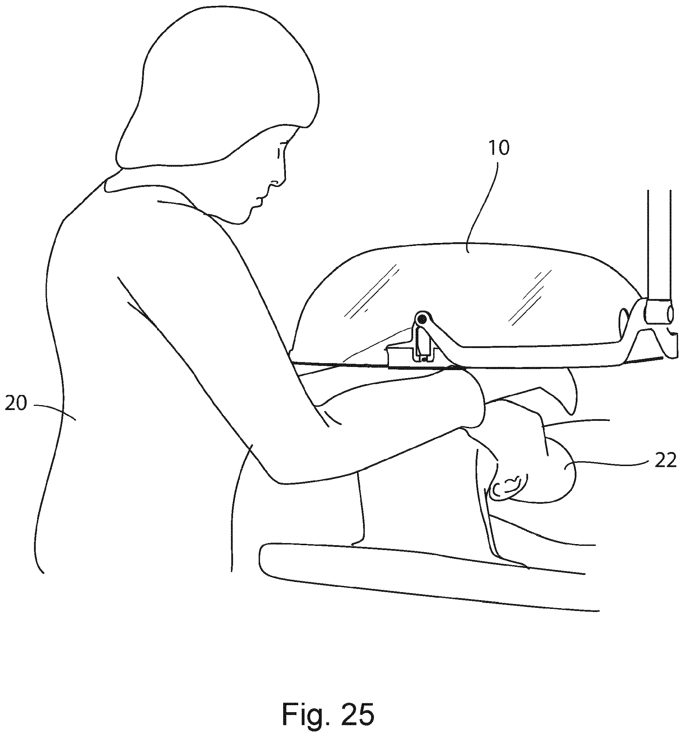

With reference to Figs. 18-25, various orientations of the shield 10 relative to the user 20 and to the patient 22 are shown. To allow for any orientation, multiple degrees of motion are enabled. The shell 12 is suspended by the articulating arm 16 and mounting assembly 18 so that its center of gravity is directly mid-position between the pivot points that allow rotational motion. This ensures that the shell 12 will not drift out of position. Once moved into position, the shield 10 stays in position until intentionally moved. When properly positioned between a patient 22 and a user 20, the shield 10 will prevent particulate matter from sneezes, coughs and from equipment from impinging upon the user 20 or the treatment room. Once properly positioned, users 20 have enough visibility and work room to carry out their tasks with their mobility not being restricted. At any time, the shield 10 can be repositioned relative to the patient 22.

The shield 10 can also include active accessories such as sensors, lights and a vacuum. The addition of sensors can monitor the efficacy of the positioning and condition of the patient 22 for example and can provide information or feedback to actively control and adjust treatment parameters. Adding lights to the shield 10 which is in close proximity to the patient 22 better illuminates the treatment area. Adding a vacuum to the shield better removes any particulates emanating from the patient 22.

The shield 10 can also be utilized for any procedure performed by a user needing a physical protection barrier such as, for example, tattoo artists, makeup artists, nail salons, ophthalmological examinations, immunization procedures or other healthcare or physical procedures or examinations. The size and shape of the shield 10 can be adapted to meet the circumstance of any specific procedure or examination.

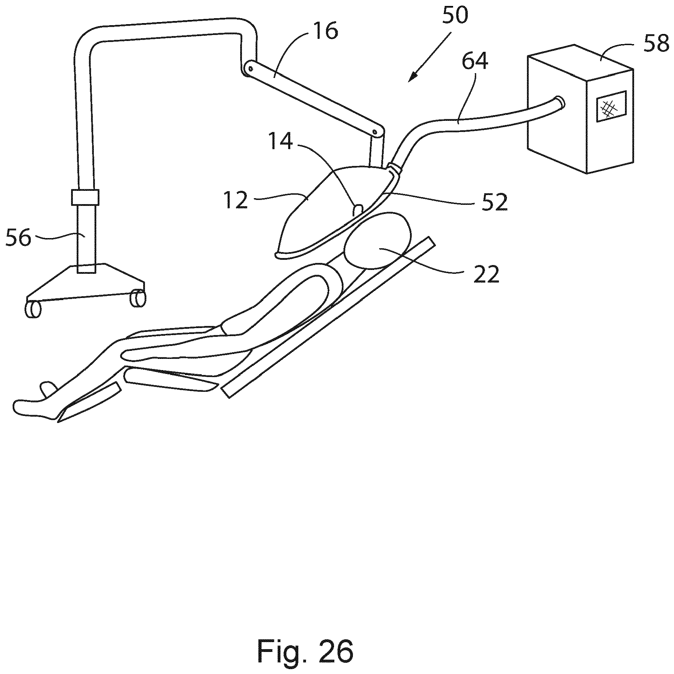

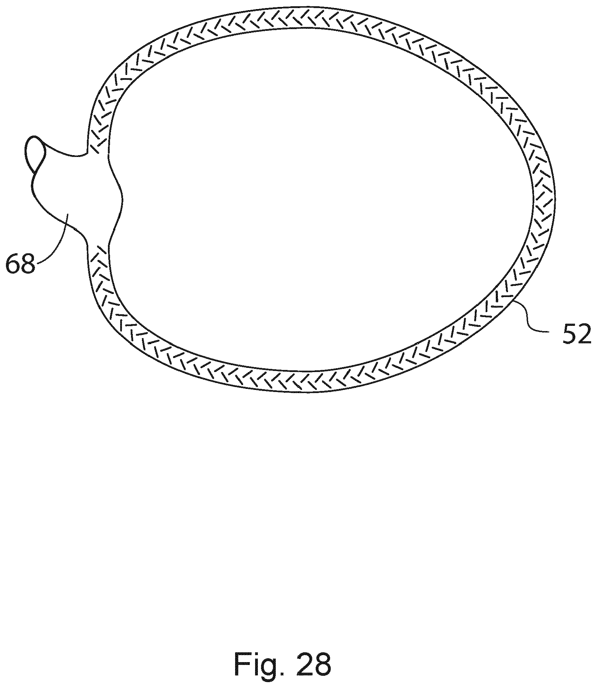

Turning now to Figs. 26-29, a second embodiment of a patient shield is shown. In this second embodiment, by adding features to a shield 50 to purposefully deflect and slow the speed of a patient cough or a sneeze, it is possible to collect a higher percentage of the biological contaminants. This is achieved by allowing the expended biological materials to collect in a baffle 52 incorporated into or added to the shield 50. A higher degree of biological contaminant collection is achieved by adding the baffle 52.

As shown in Figs. 26, the shield 50 is in use with a patient 22. As with the previously described shield 10, the shield 50 includes the shell 12, the bracket 14, and the articulating arm 16, however, different or other components can also be utilized. An alternative mounting assembly 56 is shown, however, the mounting assembly 18 of the first embodiment can also be utilized. The shield 50 further optionally includes a vacuum generator 58.

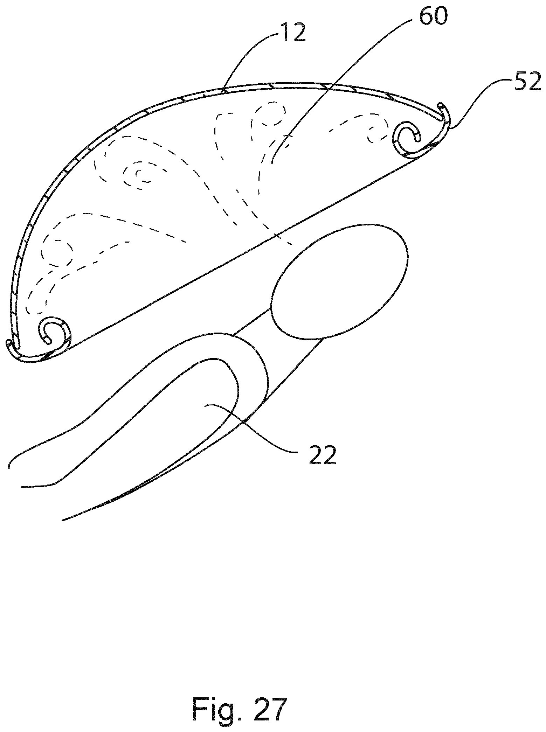

In Fig. 27, the shell 12 is shown positioned over a coughing patient 22 with biological particulate matter 60 emanating from the patient 22.

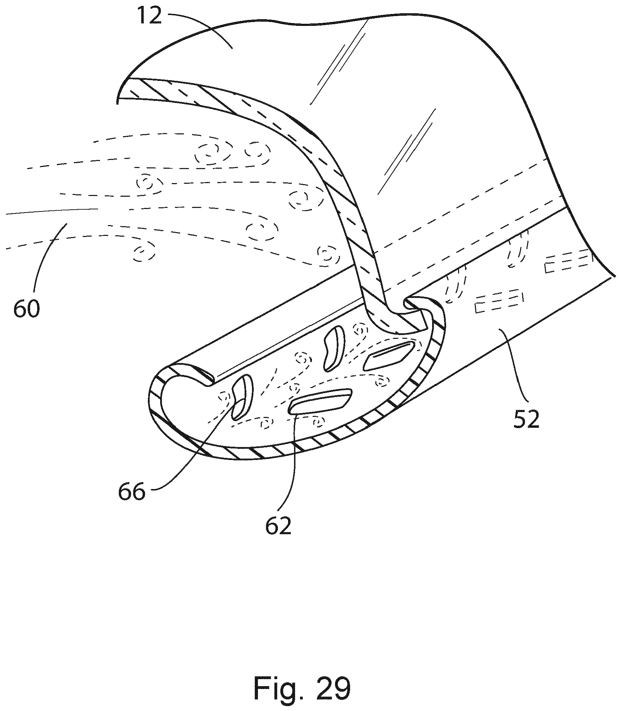

As shown in Figs. 27 and 29, the baffle 52 slows the emanating material 60 and directs it away from users 20 (not shown) and to an intentional containment area 62 rather than being dispersed around the facility or room.

As shown in Figs. 26-29, the baffle 52 is sized and positioned to gradually reduce the velocity of the material 60 until it can allow the particulates to be effectively trapped or removed by a vacuum produced by a vacuum generator 58 that is connected to the shell 12 by hose 64.

As particularly shown in Fig. 29, the baffle 52 provides an area 66 around the periphery of the shell 12 that allows the matter 60 to enter then gradually change its direction to ensure the matter 60 is not reflected back into the shell 12 and does not escape into the treatment room. Within this peripheral area 66, the vacuum from the vacuum generator 58 removes the matter 60 via outlet 68.

In an alternate embodiment, the baffle 52 can also be used with no vacuum such that particulate matter is trapped by the baffle 52.

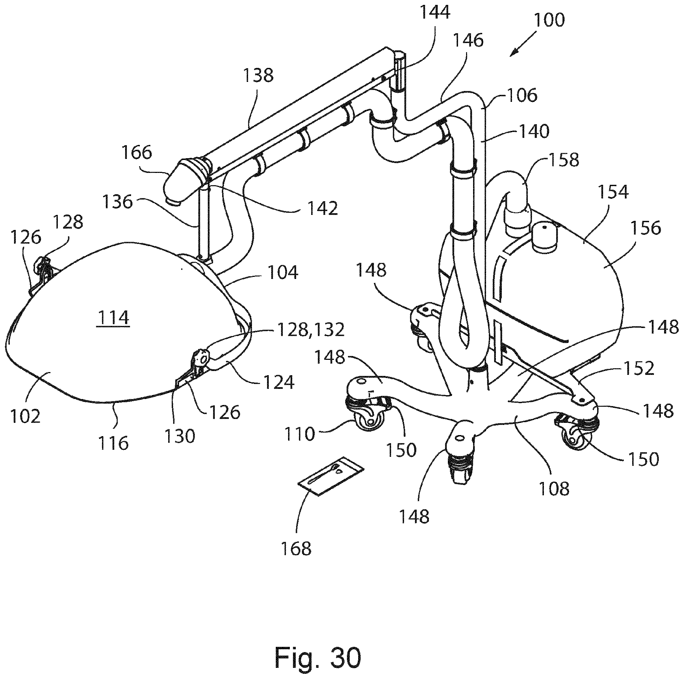

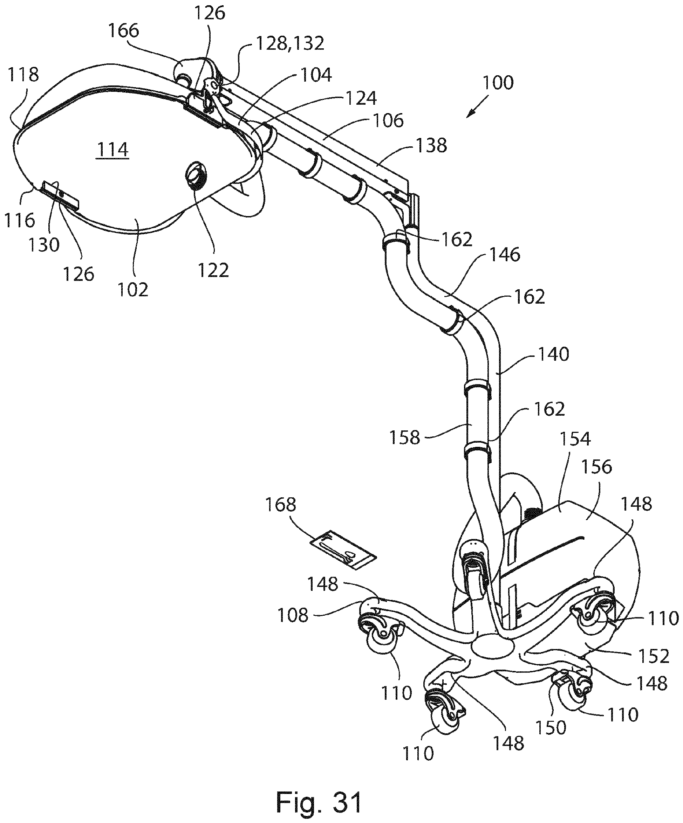

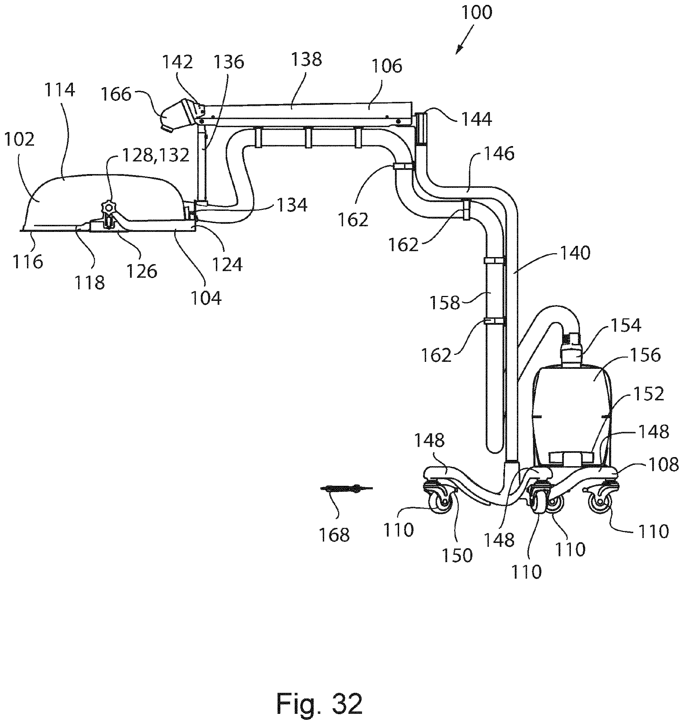

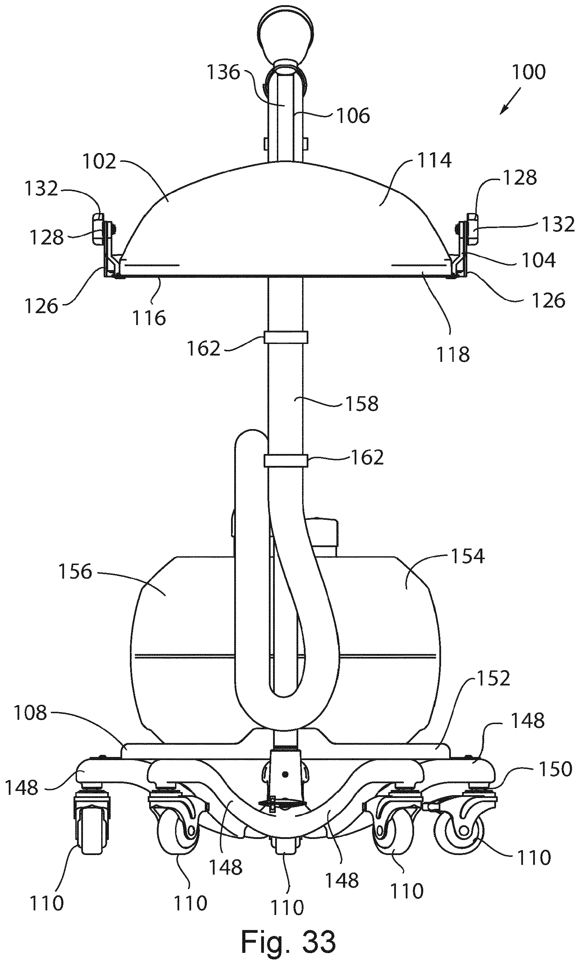

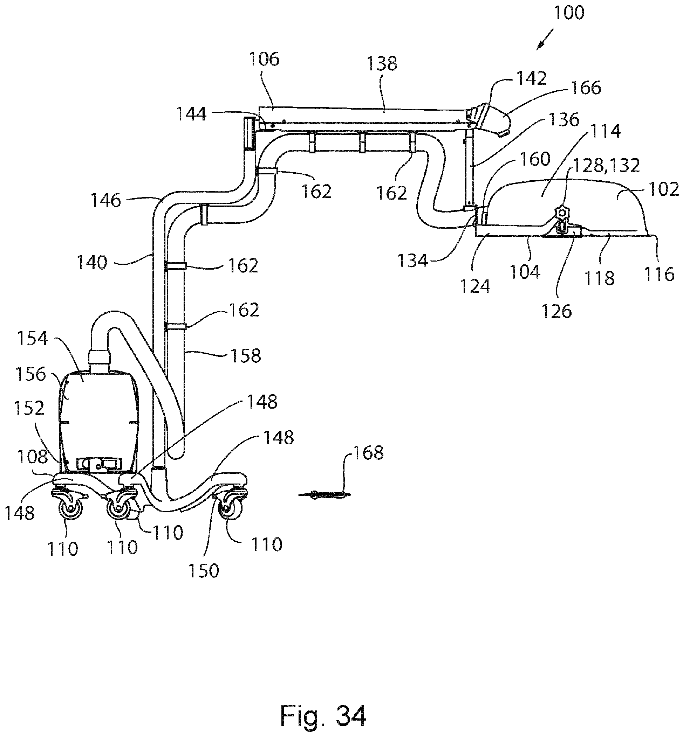

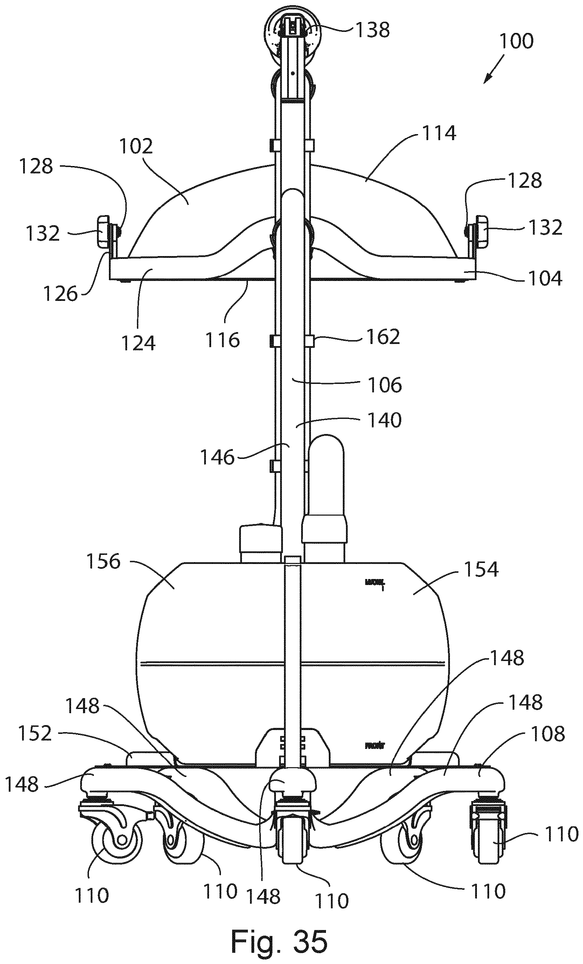

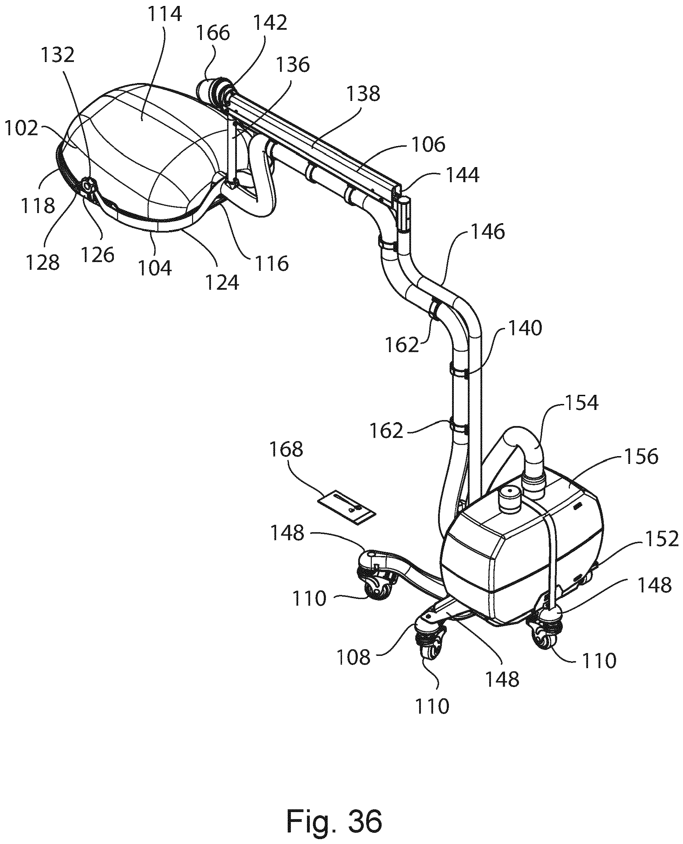

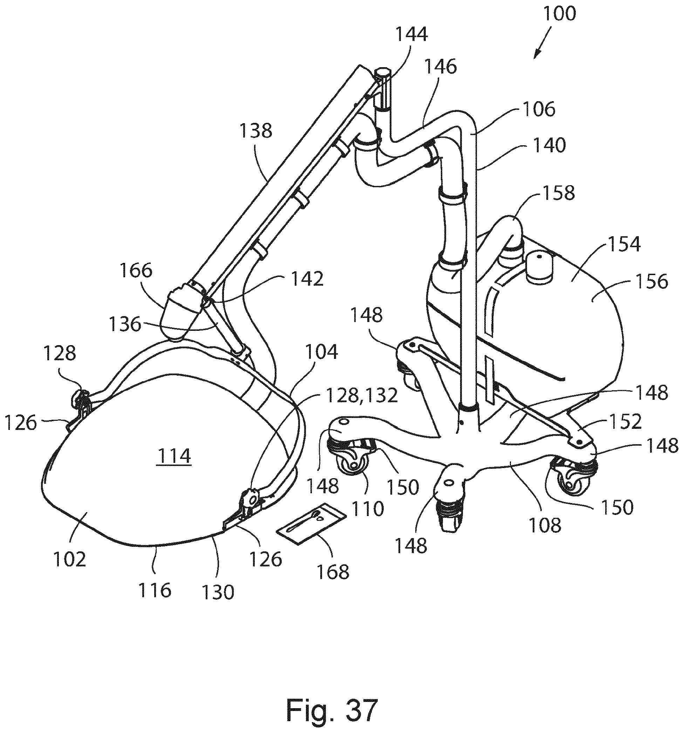

Turning now to a third embodiment of a patient shield as shown in FIGS. 30-37, the patient shield 100 includes a barrier 102 and a mounting assembly 104 that is suspended by an articulating arm assembly 106 which is secured to a mounting base 108. The mounting base 108 includes medical grade casters 110 that allow the patient shield 100 to be moved and positioned as needed during use and during storage. The mounting base 108 can alternately be secured to a location or surface such as a ceiling, a wall, a floor, a cabinet, an accessory cart, a self-contained cart, a chair or other nearby structure such as described above and shown with respect to the first and second embodiments. Preferably, the casters 110 have a non-marking tread such as made from polyurethane, for example.

The barrier 102 is preferably manufactured from clear optical grade polycarbonate plastic such as is known in the art to provide excellent visibility of the patient underneath; however, other materials can also be utilized such as glass, acrylic, other synthetic polymers, screen material or a curtain of air for example. The barrier 102 preferably is curved such as dome-shaped, however, other shapes or configurations can also be utilized. The barrier 102 preferably includes a dome portion 114, a peripheral edge 116, a flange 118 adjacent the peripheral edge 116, a pair of mounting apertures 120 (hidden from view) and a vacuum aperture 122.

The mounting assembly 104 includes a generally u-shaped bracket 124, a pair of mounting arms 126 and a pair of rotation mechanisms 128. The bracket 124, the arms 126 and the rotation mechanism 128 are secured together on each side of the barrier 102 at the mounting aperture 120. The mounting arms 126 have a channel 130 that houses the edge 116 of the barrier 102. The rotation mechanism 128 preferably includes a wheel 132. Rotation of the wheel 132 enables a user to move and hold the barrier 102 in a desired position. It should be noted that other ways and methods of securing the barrier 102 to the mounting assembly 104 for movement can also be utilized. The function of the mounting assembly 104 can also be integrated into the barrier 102 so that the articulating arm assembly 106 is securable directly to the barrier 102. The mounting assembly 104 is preferably fabricated from powder coated steel, however, other materials can also be utilized.

The mounting assembly 104 is attached to the articulating arm assembly 106 and, preferably, is attached with fasteners 134 which attach the bracket 124 to the articulating arm assembly 106.

The articulating arm assembly 106 preferably includes segments such as the illustrated three segments 136, 138 and 140 which in combination allow the barrier 102 to be freely moved into multiple orientations as need with respect to a patient and the user. The articulating arm assembly 106 is preferably manufactured from powder coated aluminum and/or powder coated steel, however, other materials can also be utilized such as plastic and titanium for example. It should also be noted that other types of articulating arms, non-segmented articulating arms and articulating arms with more or less than three segments can also be utilized.

The segment 136 is moveably secured to segment 138 using a pivot mechanism 142. Segment 138 is pivotally secured to segment 136 using pivot mechanism 144. Segment 138 includes a curved portion 146 and is secured to mounting base 108.

The mounting base 108 preferably includes five arms 148 with each arm 148 having a caster 110 attached thereto. At least one caster 110 preferably includes a locking/unlocking mechanism 150. The mounting base 108 includes a mounting platform 152. The mounting base 108 provides a low center of gravity for the patient shield 100 to minimize the changes of the patent shield 100 tipping over. The mounting base 108 is preferably fabricated from powder coated steel, however, other materials could also be utilized.

The shield 100 preferably includes a vacuum generating assembly 154 including a vacuum generator 156 supported on the platform 152, a vacuum hose 158 and a mounting bracket 160 securing the hose in the vacuum aperture 122 on the barrier 102. Optionally, ties 162 are utilized to position the hose 158 close to the articulating arm assembly 106. Alternately, the vacuum generator can be adjacent the barrier 102 which eliminates the need for a vacuum hose 158. The vacuum generating assembly 154 preferably includes a medical grade hepa filter 164 (hidden from view) such as is known in the art. The vacuum generator 156 is commercially available for medical devices as is known in the art.

The shield 100 preferably includes a light 166 positioned near the barrier 102 such as, for example, on the end of the segment 138 of the articulating arm assembly 106. The light can be any type of light given the lighting requirements for a given procedure or treatment room.

A baffle can also be utilized with the shield 100 as described above with respect to the second embodiment. A vacuum controller, such as foot pedal 168, can be utilized to control the vacuum generator 156 and/or light 166. The patient shield 100 is preferably cordless and operates using battery power for example, however, other power sources can be utilized.

The patient shield 100 can be moved and relocated by a user using one hand and held in position with the locking/unlocking mechanism 150 on the casters 110. To allow for any orientation, multiple degrees of motion of the barrier 102 are enabled. Once moved into position, the barrier 102 stays in position until intentionally moved. When properly positioned between a patient and a user, the shield 100 creates a treatment zone and will prevent aerosol and/or particulate matter from impinging upon the user or the treatment room. Aerosol and particulate matter may include saliva, blood, breath, bodily debris, splatter and the like. Once properly positioned, a user has clear visibility of the patient and the work room to carry out tasks with their mobility and access to the patient not being restricted. At any time, the shield 100 can be repositioned relative to the patient.

The shield 100 can be utilized for any procedure performed by a user needing a physical protection barrier such as, for example, tattoo artists, makeup artists, nail salons, ophthalmological examinations, immunization procedures or other healthcare or physical procedures or examinations. The size and shape of the shield 100 can be adapted to meet the circumstance of any specific procedure or examination.

The patient shield 100 is designed to contain and evacuate aerosols and particulate matter from the treatment zone and is 93% effective at doing so. The vacuum generator 156 and hepa filter 164 provide a continual evacuation and filtering to maintain a high level of hygiene and provide evacuation of any air borne viruses from the treatment zone and surrounding patient care areas. The sound produced by the patient shield 100 in operation is designed to be low to be unobtrusive to the user and patient.

The barrier 102 can be sanitized in place or can be easily removed to be sanitized elsewhere.

Various features and advantages of the invention are set forth in the following claims.

When used in this specification and the claims, the term "comprises" and "comprising" and variations thereof mean that specified features, steps or integers and included. The terms are not to be interpreted to exclude the presence of other features, steps or compounds.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilized for realising the invention in diverse forms thereof.

### PREFERRED FEATURES OF THE INVENTION

1. A shield for protecting a user from aerosol and particulate matter emanating from a patient or from equipment used on the patient, said shield comprising:
   a transparent barrier adjustably positionable adjacent a patient;
   an articulating arm secured to the barrier for adjusting the barrier in multiple orientations relative to the patient; and
   a mounting base secured to the articulating arm.
2. The shield of clause 1 wherein the barrier is dome-shaped.
3. The shield of clause 1 where the articulating arm is segmented into at least two segments.
4. The shield of clause 1 wherein the mounting base includes at least one caster.
5. The shield of clause 1 wherein the mounting base is secured to a surface.
6. The shield of clause 1 wherein the barrier is adjustably positionable by a user using only one hand.
7. The shield of clause 1 and further including a vacuum generator, a hepa filter and a light.
8. The shield of clause 1 and further including a vacuum generator and a baffle in communication with the barrier for slowing down and containing the aerosol and particulate matter so that the aerosol and particulate matter can be removed from the baffle by the vacuum generator.
9. A shield for protecting a user from aerosol and particulate matter emanating from a patient or from equipment, said shield comprising:
   a transparent barrier adjustably positionable adjacent a patient;
   an articulating arm securable to the barrier to enable the barrier to be adjustable and repositionable in multiple orientations relative to the patient;
   a vacuum generator for creating a vacuum adjacent the barrier to remove aerosol and particulate matter emanating from the patient or from equipment; and
   a mounting base.
10. The shield of clause 9 wherein the barrier is fabricated of medical grade optical polycarbonate plastic.
11. The shield of clause 9 where the articulating arm is segmented into at least three segments.
12. The shield of clause 9 wherein the mounting base includes at least three locking casters.
13. The shield of clause 9 wherein the mounting base is secured to a fixed surface.
14. The shield of clause 9 and further including a hepa filter.
15. The shield of clause 9 and further including a baffle in communication with the barrier for slowing down and containing aerosol and particulate matter so that the aerosol and particulate matter can be removed from the barrier by the vacuum generator.
16. A shield for protecting a user from aerosol and particulate matter emanating from a patient or from equipment, said shield comprising:
   a dome-shaped barrier adjustably positionable adjacent a patient;
   a segmented articulating arm secured to the barrier and enabling the barrier to be adjustable and repositionable in multiple orientations relative to the patient;
   a mounting base including locking casters;
   a vacuum generator supported by the mounting base;
   a vacuum hose in communication with the vacuum generator and the barrier;
   a light; and
   a hepa filter.
17. The shield of clause 16 and further including a baffle in communication with the barrier for slowing down and containing the aerosol and particulate matter so that the aerosol and particulate matter can be removed from the barrier by the vacuum generator.
18. The shield of clause 16 and further including a foot pedal to control at least one of the vacuum generator and the light.
19. The shield of clause 16 and including a center of gravity that is low enough to minimize the patient shield from tipping.
20. The shield of clause 16 wherein the barrier is removable from the articulating arm so as to enable sanitization of the barrier separate from the remainder of the patient shield.

## Claims

1. A shield for protecting a user from aerosol and particulate matter emanating from a patient or from equipment used on the patient, said shield comprising:
a transparent barrier adjustably positionable adjacent a patient;
an articulating arm secured to the barrier for adjusting the barrier in multiple orientations relative to the patient; and
a mounting base.

2. The shield of claim 1 wherein the barrier is dome-shaped.

3. The shield of claim 1 or 2 where the articulating arm is segmented into at least two segments.

4. The shield of any preceding claim, wherein the mounting base includes at least one caster, and optionally includes at least three locking casters.

5. The shield of any preceding claim, wherein the mounting base is secured to a surface.

6. The shield of any preceding claim, wherein the barrier is adjustably positionable by a user using only one hand.

7. The shield of any preceding claim, and further including a vacuum generator, a hepa filter and a light.

8. The shield of any preceding claim, and further including a vacuum generator and a baffle in communication with the barrier for slowing down and containing the aerosol and particulate matter so that the aerosol and particulate matter can be removed from the baffle by the vacuum generator.

9. The shield of any one of claims 1 to 7, further comprising
a vacuum generator for creating a vacuum adjacent the barrier to remove aerosol and particulate matter emanating from the patient or from equipment.

10. The shield of any preceding claim wherein the barrier is fabricated of medical grade optical polycarbonate plastic.

11. The shield of any preceding claim where the articulating arm is segmented into at least three segments.

12. The shield of any preceding claim, wherein the mounting base is secured to the articulating arm.

13. The shield of claim 7 or any claim dependent thereon, and further including a foot pedal to control at least one of the vacuum generator and the light.

14. The shield of any preceding claim and including a center of gravity that is low enough to minimize the patient shield from tipping.

15. The shield of any preceding claim wherein the barrier is removable from the articulating arm so as to enable sanitization of the barrier separate from the remainder of the patient shield.
